(19) Europäisches Patentamt
European Patent Office
Office européen des brevets

(11) Veröffentlichungsnummer: **0 064 685**
**A1**

# (12) EUROPÄISCHE PATENTANMELDUNG

(21) Anmeldenummer: **82103691.0**

(22) Anmeldetag: **30.04.82**

(51) Int. Cl.³: **C 07 D 221/18**
A 61 K 31/47
//C07D217/04

(30) Priorität: **09.05.81 DE 3118521**

(43) Veröffentlichungstag der Anmeldung:
**17.11.82 Patentblatt 82/46**

(84) Benannte Vertragsstaaten:
**AT BE CH DE FR GB IT LI LU NL SE**

(71) Anmelder: **GÖDECKE AKTIENGESELLSCHAFT**
**Salzufer 16**
**D-1000 Berlin 10(DE)**

(72) Erfinder: **Fritschi, Edgar, Dr.**
**Am Scheuerwald 2**
**D-7811 St. Peter(DE)**

(72) Erfinder: **Heldt, Wolfgang, Dr.**
**Weidenmattenstrasse 4**
**D-7830 Emmendingen 16(DE)**

(72) Erfinder: **Hartenstein, Johannes, Dr.**
**Fohrenbühl 23**
**D-7801 Stegen-Wittental(DE)**

(72) Erfinder: **Satzinger, Gerhard, Dr.**
**Im Mattenbühl 7**
**D-7809 Denzlingen(DE)**

## (54) Dibenzo(de,g)chinolin-Derivate, Verfahren zu deren Herstellung und diese enthaltende Arzneimittel.

(57) Die Anmeldung betrifft Verbindungen der allgemeinen Formel I

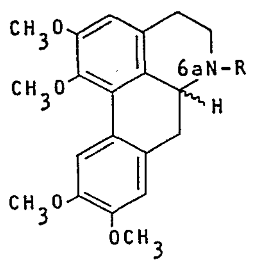

(1),

in der
R eine ungesättigte Alkylgruppe mit bis zu 6 Kohlenstoffatomen oder einen Cycloalkylalkylrest mit 4 bis 6 Kohlenstoffatomen bedeutet
sowie deren pharmakologisch verträgliche Salze, die eine gute antitussive, anlalgetische, antiallergische und und neuroleptische Wirkung aufweisen. Weiterhin hemmen sie die Blutplättchen-Aggregation. Sie können in racemischer und optisch-aktiver Form vorliegen. Die Herstellung erfolgt mittels Analogieverfahren.

EP 0 064 685 A1

## Beschreibung

Die Erfindung betrifft Dibenzo(de,g) chinolin-Derivate der allgemeinen Formel I

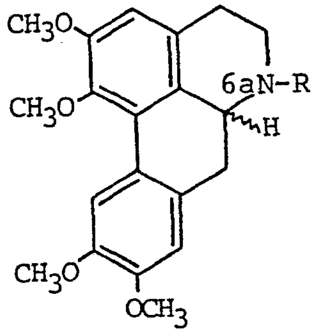

(I),

in der

R eine ungesättigte Alkylgruppe mit bis zu 6 Kohlenstoffatomen oder einen Cycloalkylrest mit 4 bis 6 Kohlenstoffatomen bedeutet

sowie deren pharmakologisch verträgliche Salze.

In der angegebenen Formel bedeutet die geschlängelte Linie an C-6a, daß Verbindungen der allgemeinen Formel I sowohl in einer racemischen Form als auch als Enantiomere auftreten können, von denen diejenigen, die eine 6aS-Konfiguration aufweisen, bevorzugt sind.

Bevorzugt sind diejenigen Verbindungen der allgemeinen Formel I, in denen R eine ungesättigte Alkylgruppe mit 3 bis 6 Kohlenstoffatomen oder einen Cycloalkylalkylrest mit 4 bis 6 Kohlenstoffatomen bedeutet. Hierunter fallen besonders der Allyl-, Methallyl-, Dimethylallyl-, 2-Butenyl-, 3-Methyl-3-butenyl-, Propargyl-, Cyclopropylmethyl-, Cyclobutylmethyl- und der Cyclopentylmethylrest.

Die Erfindung betrifft weiterhin ein Verfahren zur Herstellung der Verbindungen der allgemeinen Formel I, das dadurch gekennzeichnet ist, daß man in an sich bekannter Weise

entweder

a) eine Verbindung der allgemeinen Formel II

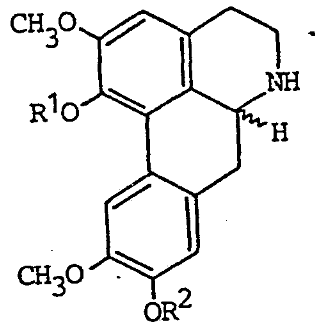

(II),

in der $R^1$ und $R^2$ gleich oder verschieden sein können und ein Wasserstoffatom oder eine Methylgruppe bedeuten N-alkyliert, und für den Fall, daß $R^1$ und/oder $R^2$ Wasserstoff ist, nachträglich O-methyliert, oder

b) eine Verbindung der allgemeinen Formel III

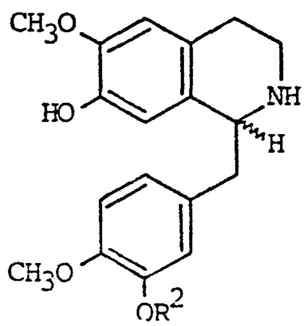

(III),

in der $R^2$ die oben genannte Bedeutung besitzt, N-alkyliert, danach cyclisiert und anschließend O-methyliert.

Die N-Alkylierung der Verbindungen II und III kann nach den für die Alkylierung sekundärer Amine bekannten Methoden erfolgen. Sie wird am einfachsten mittels reaktiver Derivate der entsprechenden Alkylverbindungen der Formel IV

X-R (IV)

in der
R die oben angegebene Bedeutung hat und X ein Halogenatom bedeutet,

2

durchgeführt. Weiterhin ist es möglich, den Rest R als Acylgruppe mit der entsprechenden Zahl von Kohlenstoffatomen durch Acylierung einzuführen und das so erhaltene Amid durch Reduktion der Carbonylgruppe in den Rest R überzuführen. Für diese Reduktion hat sich die Verwendung von komplexen Hydriden wie z.B. Lithiumaluminiumhydrid oder Natrium-dihydrido-bis-(2-methoxy-ethoxy)-aluminat bewährt. Dabei werden außer der Amidcarbonylgruppe auch eventuell vorhandene Phenolestergruppen, die bei der Acylierung phenolischer Amine gebildet werden unter Rückbildung der freien Phenole, mit reduziert.

Die Cyclisierung der Tetrahydroisochinoline der allgemeinen Formel III und von deren N-Alkyl- sowie N-Acylderivaten erfolgt nach den üblichen Verfahren der oxidativen Phenolkupplung. Besonders vorteilhaft eignet sich erfindungsgemäß die oxidative Cyclisierung von 7-Hydroxytetrahydroisochinolinen mit Vanadiumoxytrichlorid nach DE-OS 27 57 281.

Die O-Methylierung der N-Alkyl- und der N-Acylnoraporphine kann nach den für die O-Alkylierung von Phenolen herkömmlichen Verfahren, z.B. Methylierung mit Diazomethan oder Dimethylsulfat erfolgen. Als besonders vorteilhaft hat sich jedoch gemäß DE-OS 27 57 335 die O-Methylierung mit basischen Phenyltrimethylammoniumverbindungen herausgestellt.

Die als Ausgangsmaterialien für die Herstellung der erfindungsgemäßen Verbindungen I dienenden Verbindungen gemäß allgemeiner Formel II sind bekannte Verbindungen. Sie können zum Beispiel durch oxidative Cyclisierung (DE-OS 27 57 281) und O-Methylierung des 1-Hydroxy-N-noraporphins oder seines N-Trifluoracetylderivats (z.B. nach dem Verfahren der DE-OS 27 57 335) oder nach J. Organic Chemistry 41, 4049 (1976) erhalten werden.

Die als Ausgangsmaterialien nach Verfahren b) verwendeten Tetrahydroisochinoline der Formel III sind ebenfalls dem Fachmann bekannte Verbindungen:
N-Norreticulin (R=H) und N-Norcodamin ($R=CH_3$).
Die Synthese von racemischem und optisch aktivem N-Norreticulin haben K.C. Rice und A. Brossi (J. Org. Chem. 1980, 45, S. 592) beschrieben. Die Herstellung von racemischem Norcodamin ist in J. Org. Chem. 1976, 41, S. 4049, Fußnote 6, veröffentlicht.

Die Aufarbeitung der Reaktionsprodukte erfolgt ebenso wie die Isolierung und Reindarstellung der Verbindungen I nach an sich bekannten Verfahren, z.B. durch Kristallisation in Form der Basen oder geeigneter Säureadditionsverbindungen, gegebenenfalls nach vorangehender chromatographischer Reinigung.

Ein weiterer vorteilhafter Weg zur Herstellung der Enantiomeren besteht darin, daß bei der Synthese nach a) oder b) von optisch-aktiven Verbindungen der allgemeinen Formel II oder von Verbindungen der allgemeinen Formel III ausgegangen wird. Ein besonders bevorzugtes Ausgangsmaterial ist die Verbindung gemäß allgemeiner Formel III, in welcher $R^2$ einen Methylrest bedeutet, da es sich mit (+)- und (-)-Anilinweinsäuren (J. Org. Chem. 1968, 33, 3993), insbesondere mit O-Bromanilinweinsäure, oder N-Acetyl-L-leucin in die optischen Antipoden spalten läßt. Gegenüber N-Norreticulin (allgem. Formel III: $R^2$ = H), für welches die optische Spaltung mit Bromanilinweinsäure in der Literatur beschrieben ist (J.Org. Chem. 1980, 45, 592), weist dieses Ausgangsprodukt den Vorteil der leichteren Herstellbarkeit aus wohlfeilen Vorstufen auf. Außerdem treten bei der oxidativen Cyclisierung zum Aporphin mit Vanadiumoxytrichlorid gemäß Verfahren b) weniger Nebenprodukte auf.

Die Verbindungen der allgemeinen Formel I können in racemischer oder optisch-aktiver Form vorliegen. Die optisch-aktiven Formen können auf an sich bekannte Weise wie fraktionierte Kristallisation der diastereomeren Salze hergestellt werden.

Die Überführung der freien Basen der allgemeinen Formel I in deren pharmakologisch verträgliche Salze erfolgt durch Neutralisation mit einer entsprechend anwendbaren organischen oder anorganischen Säure, wie z.B. Salzsäure, Schwefelsäure, Phosphorsäure, Bromwasserstoffsäure, Essigsäure, Fumarsäure, Oxalsäure, Milchsäure, Zitronensäure, Äpfelsäure, Salicylsäure, Malonsäure, Maleinsäure, Bernsteinsäure, Weinsäure, oder Ascorbinsäure. Zur Herstellung von Arzneimitteln werden die Wirkstoffe mit üblichen Zusätzen und flüssigen oder festen Trägerstoffen verarbeitet. Die Verbindungen der Formel I können in weiten Dosierungsgrenzen in flüssiger oder fester Form oral oder parenteral appliziert werden.

Übliche Zusätze für flüssige Formen sind z.B. Tartrat- und Citrat-Puffer, Äthanol, Komplexbildner (wie Äthylendiamintetraessigsäure und deren nichttoxische Salze) sowie hochmolekulare Polymere (wie flüssiges Polyäthylenoxid) zur Viskositätsregulierung. Feste Trägerstoffe sind z. B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglycol); für orale Applikation geeignete Zubereitungen können gewünschtenfalls Geschmacks- und Süßstoffe enthalten.

Die Verbindungen der allgemeinen Formel I haben wertvolle pharmakologische Eigenschaften. Insbesondere weisen sie antitussive, analgetische, antiallergische und neuroleptische

Wirkungen auf und hemmen die Blutplättchen-Aggregation. Dabei übertreffen die Verbindungen die bekannte antitussive Wirkung des 5,6,6a,7-Tetrahydro-1,2,9,10-tetramethoxy-5-methyl-4H-dibenzo(de,g)chinolins (Glaucin) erheblich bezüglich Wirkungsstärke und Wirkungsdauer. So zeigt die Verbindung des Beispiels 1 im Hustenversuch nach Domenjoz (Arch. f. Pathol. u. Pharmakol. 215, 19 (1952)) an der Katze bei intravenöser Gabe von 1 mg/kg eine 20 Minuten dauernde Hustenhemmung, während die Standardsubstanz Glaucin bei einer Dosis von 2 mg/kg i.v. nur eine 10 Minuten dauernde Hustenhemmung ergibt.

| Verbindung | Dosis und antituss. Effekt im Versuch an der Katze | Tox. Maus $LD_{50}$ in mg/kg |
|---|---|---|
| Glaucin | 2,0 mg/kg i.v.: 10min Hustenhemmung | 58,3 iv. |
| | 10,0 " " sc. : keine " " | 20,0 sc. |
| | 4,0 " " id.: 40min " " | 530,6 ig. |
| Verbindung Beispiel 1 | 1,0 " " iv. : 20min " " | 150,0 iv. |
| | 6,25" " sc.: 40 " " " | 150,0 sc. |
| | 6,25" " id : 90 " " | ca. 1200,0 ig. |

Die perorale Einzeldosis der Verbindungen I dürfte je nach Indikation im Bereich von 30 mg bis 200 mg liegen, die bis zu dreimal täglich gegeben werden kann.

Die folgenden Beispiele dienen der weiteren Erläuterung der Erfindung:

## Beispiel 1

(+)-6-Allyl-5,6,6a,7-tetrahydro-1,2,9,10-tetramethoxy-4H-dibenzo(de,g)-chinolin

Variante 1:

3,6, g (11.4 mmol) (-)-7-Hydroxy-1-(3-hydroxy-4-methoxybenzyl)-6-methoxy-1,2,3,4-tetrahydroisochinolin (J. Org. Chem. 1980, 45, 592) in 70 ml Ethanol werden unter Rühren mit 0.88 ml (1.26 g, 10.4 mmol) Allylbromid und 2.3 g (27.4 mmol) Natriumhydrogencarbonat versetzt. Man läßt das Gemisch 30 Minuten bei Raumtemperatur rühren und erhitzt danach 3 Stunden auf 80°C. Nach Abkühlen wird im Vakuum eingedampft und der Rückstand zwischen Wasser und Methylenchlorid verteilt. Übliche Aufarbeitung und Chromatographie an Kieselgel mit Chloroform als Eluens liefern 3.3 g (+)-2-Allyl-7-hydroxy-1-(3-hydroxy-4-methoxybenzyl)-1,2,3,4-tetrahydroisochinolin als DC-einheitlichen Schaum.

MS: m/e 355 ($M^+$) base peak m/e 218
$(\alpha)_D^{RT}$ + 83 ° (c=1, $CHCl_3$)

Die gesamte Menge des erhaltenen Produkts wird unter Kühlung mit Eiswasser und Feuchtigkeitsausschluß in einem Gemisch von 30 ml Trifluoressigsäure und 30 ml absolutem Methylenchlorid gelöst. Zu dieser Lösung läßt man unter Rühren bei -10°C in einer Inertgasatmosphäre die Lösung von 1.33 ml $VOCl_3$ (2.45 g, 14.12 mmol) in 30 ml absolutem Methylenchlorid zutropfen innerhalb von ca. 5 Minuten. Man läßt das Gemisch noch 10 Minuten bei -10°C und 1/2 Stunde bei 0°C rühren. Danach wird bei Raumtemperatur am Rotationsverdampfer eingeengt und der Rückstand zwischen Eiswasser und Chloroform verteilt. Aus der Chloroformphase wird die Base mit verdünntem Ammoniak freigesetzt. Nach Aufarbeitung und Kristallisation aus Chloroform/Ether erhält man (+)-6-Allyl-5,6,6a,7-tetrahydro-1,9-dihy-

droxy-2,10-dimethoxy-4H-dibenzo(de,g)chinolin vom Schmp. 161-71°C.

Ms: m/e 353 ($M^+$)

$(\alpha)_D^{RT}$ + 34.6° (c= 0,5, MeOH)

1.69 g (4.78mmol) (+)-6-Allyl-5,6,6a,7-tetrahydro-1,9-dihydroxy-2,10-dimethoxy-4H-dibenzo(de,g)chinolin werden in 100 ml eines Gemisches von Toluol und Dimethylformamid (9:1,v/v) gelöst und auf 100°C erhitzt. Dazu läßt man innerhalb von ca. 10 Minuten 17 ml einer 1N-Lösung von Phenyltrimethylammoniumhydroxid in Methanol unter kräftigem Rühren und gleichzeitigem Abdestillieren des gebildeten Methanol/Toluol-Azeotrop zutropfen. Nach beendigter Zugabe wird das Reaktionsgemisch erhitzt, bis die Temperatur des übergehenden Destillats etwa 111°C erreicht hat. Man erwärmt noch 1 Stunde unter Rückfluß. Ist nach DC-Analyse noch Ausgangsmaterial vorhanden, wird eine weitere Portion Methylierlauge zugegeben und wie oben beschrieben verfahren. Zur Isolierung des Produkts wird filtriert und das Filtrat im Vakuum eingedampft. Nach Verteilung zwischen Wasser und Methylenchlorid und üblicher Aufarbeitung wird das Rohprodukt an Kieselgel mit Chloroform als Eluens chromatographiert und mit äthanolischem HCl ins Hydrochlorid übergeführt.

Schmp.: 214-220°C

$C_{23}H_{27}NO_4HCl$ 1/4 HO

| | | | | |
|---|---|---|---|---|
| Ber: | C 65.40 | H 6.80 | Cl 8.39 | N 3.32 |
| Gef: | 65.31 | 6.81 | 8.64 | 3.33 |

$(\alpha)_D^{22}$ + 118.6° (c=0,5, $CHCl_3$)

Das als Zwischenprodukt anfallende (+)-6-Allyl-5,6,6a,7-te-

trahydro-1,9-dihydroxy-2,10-dimethoxy-4H-dibenzo(de,g)chinolin kann auch wie folgt hergestellt werden:

582 mg (1.85 mmol) (+)-5,6,6a,7-Tetrahydro-1,9-dihydroxy-2,-10-dimethoxy-4H-dibenzo(de,g)chinolin werden in 10 ml Dimethylformamid gelöst.Nach Zusatz von 385 mg (4.58mmol) Natriumhydrogencarbonat und 0.2 ml (279.6 mg, 2.31 mmol) Allylbromid wird das Reaktionsgemisch 30 Minuten bei Raumtemperatur gerührt und danach 2 Stunden unter Rückfluß erhitzt. Anschließend wird das Lösungsmittel im Vakuum eingedampft und der Rückstand zwischen Chloroform und Wasser verteilt. Nach Aufarbeitung und Kristallisation aus Chloroform/Ether erhält man (+)-6-Allyl-5,6,6a,7-tetrahydro-1,9-dihydroxy-2,10-dimethoxy-4H-dibenzo(de,g)chinolin vom Schmp. 161 - 171°C.

<u>Variante 2:</u>
4.5 g (12.2 mmol) (+)-6-Allyl-5,6,6a,7-tetrahydro-1-hydroxy-2,9,10-trimethoxy-4H-dibenzo(de,g)chinolin werden in 230 ml eines Gemischs von Toluol und Dimethylformamid (9:1,v/v) mit 20 ml einer 1N-Lösung von Phenyltrimethylammmoniumhydroxid in Methanol wie in Variante 1 beschrieben umgesetzt. Nach entsprechender Aufarbeitung erhält man (+)-6-Allyl-5,6,6a,7-tetrahydro-1,2,9,10-tetramethoxy-4H-dibenzo(de,g)-chinolin, das sich in jeder Hinsicht mit dem nach Variante 1 gewonnenen Produkt als identisch erweist.

$(\alpha)_D^{RT}$ (Hydrochlorid): + 117.7° (c=0.5, Methanol)

Das als Ausgangsprodukt verwendete (+)-6-Allyl-5,6,6a,7-tetrahydro-1-hydroxy-2,9,10-trimethoxy-4H-dibenzo(de,g)chinolin wird wie folgt hergestellt:

<u>a) Racemattrennung von 7-Hydroxy-6-methoxy-1-(3,4-dimethoxybenzyl)-1,2,3,4-tetrahydroisochinolin via Bromanilintartrat</u>

77 g (0.233 Mol) ($\pm$)-7-Hydroxy-6-methoxy-1-(3,4-dimethoxybenzyl)-1,2,3,4-tetrahydroisochinolin (N-Norcodamin; Herstellung: J.Org. Chem. 1976, 41, 4049) werden bei ca. 50°C in 2oo ml Methanol gelöst und mit einer Lösung von 75.9 g (0.235 Mol) (+)-2'-Bromanilinweinsäure-Hydrat (J.Org.Chem. 1968, 33, 3993) in 200 ml Methanol versetzt. Man läßt das Gemisch 2 Tage bei Raumtemperatur stehen und saugt dann die farblosen bis gelblichen Kristalle ab.

Schmp. 166-72°C

$(\alpha)_D^{RT}$ + 38.7° (c=0.5, Methanol)

Die aus dem Salz in üblicher Weise freigesetzte Base kristallisiert aus Ethanol. Schmp. 156-59°C.

$(\alpha)_D^{RT}$ - 40.6° (c=0.5, Methanol)

MS: m/e 329 ($M^+$), base peak m/e 178

b) Racemattrennung via N-Acetyl-L-leucinat

18.9 g (57.4 mmol) (±)-7-Hydroxy-6-methoxy-1-(3,4-dimethoxybenzyl)-1,2,3,4-tetrahydroisochinolin werden bei 60°C in 70ml Methanol gelöst und mit einer ebenfalls auf 60°C erwärmten Lösung von 9.94 g (57.4 mmol) N-Acetyl-L-leucin in 50 ml Methanol versetzt. Die erhaltene Lösung wird durch Eindampfen im Vakuum konzentriert. Nach Zugabe von Isopropanol/Ether und Rekristallisation aus demselben Lösungsmittelgemisch erhält man das N-Acetyl-L-leucinat von(-)-7-Hydroxy-6-methoxy-1-(3,4-dimethylbenzyl)-1,2,3,4-tetrahydroisochinolin vom Schmp. 179-86°C.

$(\alpha)_D^{RT}$ - 21.5 ± 1° (c= 0.5, $H_2O$)

Überführen in die Base und Kristallisation aus Ethanol liefern die Titelverbindung vom Schmp. 156-9°C.

$(\alpha)_D^{RT}$ -37.3° (c=0.5, MeOH)

27.7. g(84 mmol) (-)-7-Hydroxy-6-methoxy-1-(3,4-dimethoxybenzyl)-1,2,3,4-tetrahydroisochinolin in 430 ml Ethanol werden mit 9.18 g( 75.9 mmol) Allylbromid und 17.6 g (209 mmol) Natriumhydrogencarbonat versetzt. Man erhitzt das Gemisch unter Rühren 3 h auf 80°C. Nach dem Abkühlen und Eindampfen i.V. wird der Rückstand zwischen Wasser und Methylenchlorid verteilt. Übliche Aufarbeitung und chromatographische Reinigung (Kieselgel; Chloroform) liefern 22.0 g eines hellgelben Schaums.

Durch Kristallisation aus Isopropanol-Ether erhält man (+)-2-Allyl-7-hydroxy-6-methoxy-1-(3,4-dimethoxybenzyl)-1,2,3,4-tetrahydroisochinolin in Form farbloser Kristalle vom Schmp. 108-112°C.

$(\alpha)_D^{RT}$ + 65.7° (c=1, $CHCl_3$)

MS: m/e 369 ($M^+$), base peak m/e 218

22 g (59.54 mmol) (+)-2-Allyl-7-hydroxy-6-methoxy-1-(3,4-dimethoxybenzyl)-1,2,3,4-tetrahydroisochinolin werden in einem Gemisch von 190 ml Trifluoressigsäure und 190 ml absolutem Methylenchlorid bei -10°C unter Feuchtigkeitsausschluß mit einer Lösung von 6.0 ml (11.04, 63.7 mmol) Vanadiumoxytrichlorid in 190 ml absolutem Methylenchlorid versetzt. Man läßt das Gemisch 30 Minuten bei -10°C, 1 Stunde bei 0°C und 2 Stunden bei Raumtemperatur rühren. Danach wird das Reaktionsgemisch durch Abdampfen der Lösungsmittel im Vakuum bei Raumtemperatur auf ca. 1/3 seines Volumens konzentriert Der Rückstand wird mit Eiswasser versetzt und mit Chloroform extrahiert. Die Chloroformphase wird mit wäßriger Ammoniaklösung basisch gestellt und in üblicher Weise aufgearbeitet. Durch Kristallisation aus Äthanol erhält man (+)-6-Allyl-5,6,6a,7-tetrahydro-1-hydroxy-2,9,10,trimethoxy-4H-dibenzo(de,g)chinolin vom Schmp. 85-93/134-8°C.

MS: m/e 367 ($M^+$)

NMR: $\delta$($CDCl_3$), 3.93 ($3xOCH_3$), 5.0-6.2 (m, 3H, $-CH{=}CH_2$)
6.56 (s, 8-H), 6.80 (s, 3-H), 8.06 (s, 11-H)

Hydrochlorid: Schmp. 192-210°C

Hydrobromid-Monohydrat: Schmp. 214-224°C

Variante 3:

1 g (1.94 mmol) (+)-N-Norglaucin•(-)-N-Acetyl-L-Leucinat ($[\alpha]_D^{20}$ +33.4° (c o.5, MeOH) werden in 25 ml Methylenchlorid aufgenommen und mit 5 %iger wässriger Natriumhydrogencarbonat-Lösung in die Base übergeführt. Der Methylenchloridextrakt wird getrocknet, im Vakuum eingedampft und der Rückstand in 20 ml absolutem Ethanol gelöst. Nach Zugabe von 400 mg (4.76 mmol) Natriumhydrogencarbonat und 0.19 ml (226 mg, 2.20 mmol) Allylbromid läßt man das Gemisch 30 Minuten bei Raumtemperatur rühren und erhitzt dann 2 Stunden unter Rückfluß. Nach dem Abkühlen wird im Vakuum eingedampft und der Rückstand zwischen Wasser und Methylenchlorid verteilt. Übliche Aufarbeitung und Kristallisation aus Ethanol liefern (+)-6-Allyl-5,6,6a,7-tetrahydro-1,2,9,10-tetramethoxy-4H-dibenzo(de,g)chinolin vom Schmp. 139-43°C.

MS: m/e 381 ($M^+$), base peak m/e 41 ($CH_2{=}CH-CH_2^+$)

IR (KBr) u.a. 920/985 $cm^{-1}$ ($-CH{=}CH_2$)

NMR (δ, $CDCl_3$) 3.63 (s, 1-$OCH_3$), 3.90 (3x$OCH_3$), 5.0-6.0 (m, 3H, -CH=$CH_2$), 6.59 (s, 8-H), 6.76 (s, 3-H), 8.06 (s, 11-H)

Durch Behandeln mit äthanolischer Salzsäure erhält man das Hydrochlorid in Form rötlicher Kristalle vom Schmp. 214-220°C.

## Beispiel 2

(-)-6-Allyl-5,6,6a,7-tetrahydro -1,2,9,10-tetramethoxy-4H-dibenzo(de,g)chinolin

8.0 g (25.37 mmol) (+)-7-Hydroxy-1-(3-hydroxy-4-methoxybenzyl)-6-methoxy-1,2,3,4-tetrahydroisochinolin (J.Org.Chem. 1980, 45, 592) in 150 ml Ethanol werden mit 5.10 g (60.7 mmol) Natriumhydrogencarbonat und 2.19 ml (3.07 g) Allylbromid analog Beispiel 1 umgesetzt. Das nach entsprechender Aufarbeitung erhaltene N-Allylierungsprodukt (8.0 g, 22.5mmol, gelblicher Schaum) wird in einem Gemisch aus 70 ml Trifluoressigsäure und 70 ml Methylenchlorid bei -10°C mit 3.2 ml (5.89 g, 33.98 mmol) Vanadiumoxytrichlorid in 35 ml absolutem Methylenchlorid analog Beispiel 1 umgesetzt. Das erhaltene Rohprodukt (8 g, Kristallisation aus Chloroform/Ether, Schmp. 159-65°C) wird analog Beispiel 1 mit 115 ml einer 1N-Lösung von Phenyltrimethylammoniumhydroxid in Methanol in einem Gemisch (400 ml, 9:1) von Toluol und Dimethylformamid O-methyliert. Das nach entsprechender Aufarbeitung erhaltene Rohprodukt wird nach chromatographischer Reinigung an Kieselgel mit Chloroform als Eluens mit ethanolischer Salzsäure in das kristalline Hydrochlorid übergeführt. Man erhält (-)-6-Allyl-5,6,6a,7-tetrahydro-1,2,9,10-tetramethoxy-4H-dibenzo-(de,g)chinolin·HCl vom Schmp. 210-17°C.

$(\alpha)_D^{RT}$ - 122° (c=0.5, $CHCl_3$)

$C_{23}H_{27}NO_4 \cdot HCl \cdot 1/4H_2O$

| | | | | | | | | |
|---|---|---|---|---|---|---|---|---|
| Ber.: | C | 65.40 | H | 6.80 | Cl | 8.39 | N | 3.32 % |
| Gef.: | | 65.26 | | 6.53 | | 8.64 | | 3.35 % |

## Beispiel 3

(+)-5,6,6a,7-Tetrahydro-1,2,9,10-tetramethoxy-6-(3-methyl-2-butenyl)-4H-dibenzo(de,g)chinolin

Methylierung von (+)-5,6,6a,7-Tetrahydro-1,9-dihydroxy-2,-10-dimethoxy-6-(3-methyl-2-butenyl)-4H-dibenzo(de,g)chinolin mit Phenyltrimethylammoniumhydroxid und Aufarbeitung analog Beispiel 1 liefern (+)-5,6,6a,7-Tetrahydro-1,2,9,-10-tetramethoxy-6-(3-methyl-2-butenyl)-4H-dibenzo(de,g)chinolin in Form des Hydrochlorids vom Schmp. 214-226°C.

MS: m/e 409 ($M^+$)

NMR: $\delta$($CDCl_3$) 1.80 (b, 6H), 3.66 ($1xOCH_3$), 3.90 ($3xOCH_3$)
5.53 (m, 1H), 6.64 (s, 8-H), 6.80 (s,3-H),
8.05 (s, 11-H)

Durch die Überführung in die Base und Umsetzung mit L-(+)-Weinsäure erhält man das kristalline L-(+)-Hydrogentartrat vom Schmp. 115-25°/165-76°C.

$C_{25}H_{31}NO_4 \cdot C_4H_6O_6$

| | | | | | | |
|---|---|---|---|---|---|---|
| Ber.: | C | 61.25 | H | 6.73 | N | 2.46 % |
| Gef.: | | 61.23 | | 6.62 | | 2.42 % |

$(\alpha)_D^{RT}$ + 81.1° (c 0.5, $CHCl_3$)

Das als Ausgangsprodukt verwendete (+)-5,6,6a,7-Tetrahydro-1,9-dihydroxy-2,10-dimethoxy-6-(3-methyl-2-butenyl)-4H-dibenzo(de,g) chinolin wird wie folgt hergestellt:

1.38 (4.37 mmol) (-)-7-Hydroxy-1-(3-hydroxy-4-methoxybenzyl)-6-methoxy-1,2,3,4-tetrahydroisochinolin in 25 ml absolutem Ethanol werden mit 0.9 g (10.7 mmol) Natriumhydrogen-

carbonat, 0.44 g (4.2 mmol) 1-Chlor-3-methyl-2-buten und 100 mg Kaliumjodid versetzt. Man läßt 1 Stunde bei Raumtemperatur und 4 Stunden bei 80°C reagieren. Übliche Aufarbeitung und chromatographische Reinigung an Kieselgel mit Chloroform als Eluens liefern (-)-7-Hydrox-1-(3-hydroxy-4-methoxybenzyl)-6-methoxy-2-(3-methyl-2-butenyl)-1,2,3,4-tetrahydroisochinolin in Form eines gelblichen Schaums.

MS: m/e 383 ($M^+$), base peak m/e 246

6.49 g (16.9 mmol) (-)-7-Hydroxy-1-(3-hydroxy-4-methoxybenzyl)-6-methoxy-2-(3-methyl-2-butenyl)-1,2,3,4-tetrahydroisochinolin werden dann in 55 ml Trifluoressigsäure und 55 ml absolutem Methylenchlorid mit 2.3 ml (4.23 g, 24.4 mmol) Vanadiumoxytrichlorid bei -10 °C analog Beispiel 1 umgesetzt. Das bei der Aufarbeitung erhaltene (+)-5,6,6a,-7-Tetrahydro-1,9-dihydro-2,10-dimethoxy-6-(3-methyl-2-butenyl)-4H-dibenzo(de,g)chinolin kann ohne weitere Reinigung für die nachfolgende Methylierung verwendet werden.

## BEISPIEL 4

(+)-5,6,6a,7-Tetrahydro-1,2,9,10-tetramethoxy-6-(2-methyl-2-propenyl-4H-dibenzo(de,g)chinolin

5.7 g (18.07 mmol) (-)-7-Hydroxy-1-(3-hydroxy-4-methoxybenzyl)-6-methoxy-1,2,3,4-tetrahydroisochinolin und 3.7 g (44 mmol) Natriumhydrogencarbonat in 120 ml Ethanol werden mit 1.76 ml (1.64 g, 18.07 mmol) 3-Chlor-2-methyl-1-propen versetzt. Man läßt 1/2 Stunde bei Raumtemperatur, danach 3 Stunden bei 80°C unter Rühren reagieren. Übliche Aufarbeitung und chromatographische Reinigung an Kieselgel mit Chloroform als Eluens liefern (+)-7-Hydroxy-1-(3-hydroxy-4-methoxybenzyl)-6-methoxy-2-(2-methyl-2-propenyl)-1,2,3,4-tetrahydroisochinolin in Form eines DC-einheitlichen gelblichen Schaums.

3.0 g (8.12 mmol) des oben erhaltenen Materials werden in einem Gemisch von 25 ml Trifluoressigsäure und 25 ml absolutem Methylenchlorid gelöst und bei -10°C im Abstand von 10 Minuten mit 2 x 0,41 ml (1.5 g, 8.80 mmol) Vanadiumoxytrichlorid analog Beispiel 1 umgesetzt. Entsprechende Aufarbeitung liefert (+)-5,6,6a,7-Tetrahydro-1,9-dihydroxy-2,10-dimethoxy-6-(2-methyl-2-propenyl)-4H-dibenzo(de,g)chinolin in praktisch quantitativer Ausbeute in Form eines DC-einheitlichen gelblichen Schaums, welcher in Toluol/Dimethylformamid mit Phenyltrimethylammoniumhydroxid analog Beispiel 1 O-methyliert wird. Entsprechende Aufarbeitung und Chromatographie des Rohprodukts an Kieselgel mit Chloroform als Eluens liefern (+)-5,6,6a,7-Tetrahydro-1,2,9,10-tetramethoxy-6-(2-methyl-2-propenyl)-4H-dibenzo(de,g)chinolin als gelblichen Schaum. Behandeln mit ethanolischer Lösung ergibt kristallines Hydrochlorid vom Schmp. 195-208°C.

MS: m/e 395 ($M^+$)

NMR: (DMSO-$d_6$) 2.0 (s, $CH_3$), 3.66 (1-$OCH_3$), 3.80-3.88 (3x$OCH_3$), 5.37 (m, 2H), 6.93 (s, 1H), 7.05 (s, 1H), 7.96 (11H)

$(\alpha)_D$ + 90.2° (c 0.5, $CHCl_3$)

L-(+)-Tartrat: Schmp. 103-118°C (aus Methanol-Isopropanol-Aceton)

$C_{24}H_{29}NO_4 \cdot C_4H_6O_6 \cdot 1\ 1/4\ H_2O$

| | | | |
|---|---|---|---|
| Ber.: | C 59.19 | H 6.65 | N 2.46 % |
| Gef.: | 59.03 | 6.49 | 2.46 % |

## B e i s p i e l 5

(+)-6-Cyclopropylmethyl-5,6,6a,7-tetrahydro-1,2,9,10-tetramethoxy-4H-dibenzo(de,g)chinolin

16.2 g (48.9 mmol) (-)-7-Hydroxy-1-(3-hydroxy-4-methoxybenzyl)-6-methoxy-1,2,3,4-tetrahydroisochinolin, 8.2 g (100 mmol) Natriumhydrogencarbonat und 500 mg Kaliumjodid werden in 200 ml abs. Ethanol gelöst und mit 4.5 ml (4.44 g, 49 mmol) Chlormethylcyclopropan versetzt. Man läßt das Gemisch 40 h unter Rückfluß in einer Inertgasatmosphäre kochen. Übliche Aufarbeitung und chromatographische Trennung von unumgesetztem Ausgangsmaterial an Kieselgel mit Chloroform als Eluens liefern (+)-2-Cyclopropylmethyl-7-hydroxy-1-(3-hydroxy-4-methoxybenzyl)-6-methoxy-1,2,3,4-tetrahydroisochinolin vom Schmp. 70-81°C (Methanol/Ether/Hexan).

MS: Molekulargewicht 383; m/e EI 232; m/e CI 384
$[\alpha]_D$ + 70.0° ( c 0.5, $CHCl_3$)

4.5 g (11.7 mmol) des oben erhaltenen Materials werden analog Beispiel 1 in einem Gemisch aus 40 ml Trifluoressigsäure und 40 ml abs. Methylenchlorid bei -10°C mit 1.2 ml (12.7 mmol) Vanadiumoxytrichlorid umgesetzt. Die entsprechende Aufarbeitung liefert (+)-6-Cyclopropylmethyl-5,6,6a,-7-tetrahydro-1,9-dihydroxy-2,10-dimethoxy-4H-dibenzo(de,g)-chinolin vom Schmp. 80 - 84°C (Ethanol/Ether).

MS: m/e 381 ($M^+$)

Methylierung des vorstehend beschriebenen Produktes mit Phenyltrimethylammoniumhydroxid und Aufarbeitung analog Beispiel 1 liefern die Titelverbindung in Form des Hydrochlorids vom Schmp. 222 - 235 °C.

MS: m/e 395 ($M^+$)

$C_{24}H_{29}NO_4 \cdot HCl \cdot 1/4H_2O$

| | | | | |
|---|---|---|---|---|
| Ber.: | C 66.05 | H 7.04 | Cl 8.12 | N 3.20 % |
| Gef.: | 65.81 | 6.89 | 7.62 | 3.00 |

$[\alpha]_D^{22}$ + 45° (0.5, $CHCl_3$)

NMR: $\delta$($CDCl_3$) 0.5-1.5 (m,5H,Cyclopropyl-H), 3.70 (1-$OCH_3$), 3.96 (3x$OCH_3$), 6.67 (s, 8-H), 6.80 (s, 3-H) 8.07 (s, 11-H)

## Beispiel 6

(±)-6-(2-Propinyl)-5,6,6a,7-tetrahydro-1,2,9,10-tetramethoxy-4H-dibenzo/de,g/chinolin

1.20 g (2.7 mmol) (±)-5,6,6a,7-Tetrahydro-1-hydroxy-2,9,-10-trimethoxy-4H-dibenzo/de,g/chinolin werden in 25 ml Ethanol gelöst und mit 560 mg Natriumhydrogencarbonat und 0.26 ml (2.95 mmol) Propargylbromid versetzt: Manläßt 1 h bei Raumtemperatur und danach 2 h bei 80 °C unter Rückfluß rühren. Übliche Aufarbeitung und Kristallisation aus Ethanol liefern (±)-6-Propargyl-5,6,6a,7-tetrahydro-1-hydroxy-2,9,-10-trimethoxy-4H-dibenzo/de,g/chinolin vom Schmp. 135 - 150 °C.

Ms: m/e 365 ($M^+$), 40 (base peak)

Hydrochlorid: Schmp. 172 - 180 °C.

Methylierung des vorstehend beschriebenen Produkts mit Phenyltrimethylammoniumhydroxid und Aufarbeitung analog Beispiel 1 liefern die Titelverbindung in Form des Hydrochlorids vom Schmp. 224 - 32 °C.

MS: m/e 379 ($M^+$), 339 (base peak)

$C_{23}H_{25}NO_4 \cdot HCl$

| | | | | | | | | |
|---|---|---|---|---|---|---|---|---|
| Ber.: | C | 66.42 | H | 6.30 | Cl | 8.52 | N | 3.37 % |
| | | 66.28 | | 6.35 | | 8.70 | | 3.29 % |

Ausgehend von optisch-aktivem 5,6,6a,7-Tetrahydro-1-hydroxy-2,9,10-trimethoxy-4H-dibenzo/de,g/chinolin erhält man in der gleichen Reaktionsfolge die entsprechenden enantiomeren Hydrochloride vom Schmp. 170 - 182 °C.

Rechtdrehendes Hydrochlorid: $[\alpha]_D^{22}$: + 161° (0.5, $CHCl_3$)

Linksdrehendes Hydrochlorid: $[\alpha]_D^{22}$: - 157° (0.5, $CHCl_3$)

Patentansprüche

1.) Dibenzo(de,g)chinolin-Derivate der allgemeinen Formel I

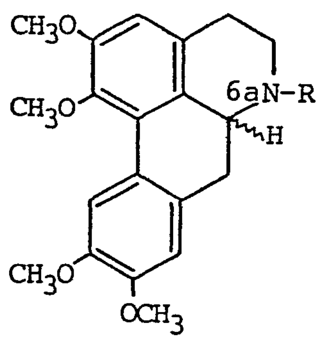

(I),

in der
R eine ungesättigte Alkylgruppe mit bis zu 6 Kohlenstoffatomen oder einen Cycloalkylalkylrest mit 4 bis 6 Kohlenstoffatomen bedeutet

sowie deren pharmakologisch verträgliche Salze

2.) Dibenzo(de,g)chinolin-Derivate gemäß Anspruch 1, dadurch gekennzeichnet, daß R eine ungesättigte Alkylgruppe mit 3 bis 6 Kohlenstoffatomen oder einen Cycloalkylalkylrest mit 4 bis 6 Kohlenstoffatomen bedeutet.

3.) Dibenzo(de,g)chinolin-Derivate gemäß Anspruch 1 und dadurch gekennzeichnet, daß R einen Allyl-, Methallyl- Dimethylallyl-, 2-Butenyl-, 3-Methyl-3-butenyl-, Propargyl-, Cyclopropylmethyl-, Cyclobutylmethyl- oder Cyclopentylmethylrest bedeutet.

4.) Verfahren zur Herstellung der Verbindungen der allgemeinen Formel I, das dadurch gekennzeichnet ist, daß

man in an sich bekannter Weise entweder

a) eine Verbindung der allgemeinen Formel II

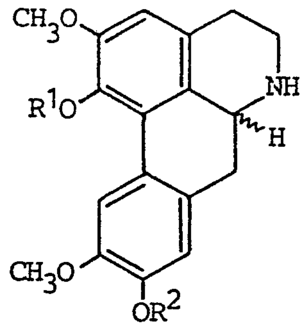

(II),

in der $R^1$ und $R^2$ gleich oder verschieden sein können und ein Wasserstoffatom oder eine Methylgruppe bedeuten N-alkyliert und für den Fall, daß $R^1$ und/oder $R^2$ Wasserstoff ist, nachträglich O-methyliert, oder

b) eine Verbindung der allgemeinen Formel III

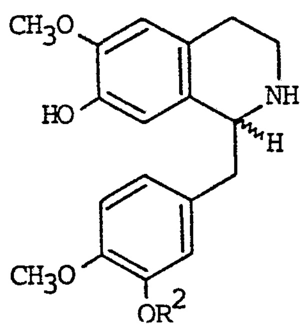

(III),

in der $R^2$ die obengenannte Bedeutung besitzt, N-alkyliert, danach cyclisiert und anschließend O-methyliert.

5.) Arzneimittel, dadurch gekennzeichnet, daß als Wirkstoff mindestens eine Verbindung gemäß Anspruch 1 enthalten ist.

0064685

Europäisches Patentamt

# EUROPÄISCHER RECHERCHENBERICHT

Nummer der Anmeldung

EP 82 10 3691

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | JOURNAL OF MEDICINAL CHEMISTRY, Band 18, Nr. 10, Oktober 1975, Seiten 1000-1003, Columbus Ohio (USA); E.R.ATKINSON et al.: "Emetic activity of N-Substituted norapomorphines". *Insgesamt* | 1-3,5 | C 07 D 221/18<br>A 61 K 31/47 //<br>C 07 D 217/04 |
| | --- | | |
| D,Y | DE-A-2 757 335 (WARNER LAMBERT) *Insgesamt* | 1,5 | |
| | --- | | |
| D,Y | JOURNAL OF ORGANIC CHEMISTRY, Band 41, Nr. 25, 1976, Seiten 4049-50, Columbus Ohio (USA); S.M.KUPCHAN et al.: "Efficient intramolecular monophenol oxidative coupling". *Insgesamt* | 1,4-5 | |
| | --- | | RECHERCHIERTE SACHGEBIETE (Int. Cl. 3) |
| P | EP-A-0 042 659 (GÖDECKE) *Insgesamt* | 1-5 | |
| | --- | | |
| P | EP-A-0 040 074 (A.D.LITTLE) *Insgesamt* | 1-3,5 | C 07 D 221/00<br>A 61 K 31/00 |
| | ----- | | |

Der vorliegende Recherchenbericht wurde für alle Patentansprüche erstellt.

| Recherchenort | Abschlußdatum der Recherche | Prüfer |
|---|---|---|
| DEN HAAG | 12-08-1982 | NUYTS A.M.K.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument